Europäisches Patentamt
European Patent Office
Office européen des brevets

(19)

(11) Veröffentlichungsnummer: **0 270 759 A2**

(12) **EUROPÄISCHE PATENTANMELDUNG**

(21) Anmeldenummer: 87114308.7

(22) Anmeldetag: 01.10.87

(51) Int. Cl.[4]: **C07C 121/48** , C07C 120/02 , C07C 49/637

(30) Priorität: 26.11.86 DE 3640306

(43) Veröffentlichungstag der Anmeldung: 15.06.88 Patentblatt 88/24

(84) Benannte Vertragsstaaten: DE FR GB IT

(71) Anmelder: HÜLS AKTIENGESELLSCHAFT
Patentabteilung / PB 15 - Postfach 13 20
D-4370 Marl 1(DE)

(72) Erfinder: Büschken, Wilfried, Dr.
Rosenkamp 10
D-4358 Haltern 6(DE)
Erfinder: Rindtorff, Klaus, Dr.
Keplerstrasse 13
D-4350 Recklinghausen(DE)

(54) **Verfahren zur Herstellung von 4.4.8.8.10-Pentamethyl-10-cyano-bicyclo-[4.4.0]-decen-(1,6)-on(2).**

(57) Zur Herstellung von 4.4.8.8.10-Pentamethyl-10-cyano-bicyclo-[4.4.0]-decen-(1,6)-on-(2) fraktioniert man das Sumpfprodukt, das bei der Herstellung von Isophoron anfällt, und isoliert einen im wesentlichen aus $C_{15}$ -Verbindungen bestehenden Ketonschnitt, der als Hauptkomponenten die Isomeren B und C enthält.

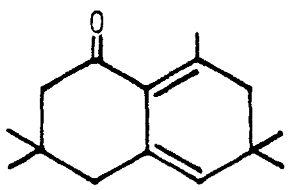

B

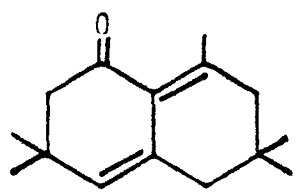

C

An diesen Ketonschnitt lagert man in Gegenwart eines basischen Stoffes bei Temperaturen von 50 bis 300 °C Cyanwasserstoff an und fraktioniert das Additionsprodukt nach einer Wäsche destillativ.

EP 0 270 759 A2

Xerox Copy Centre

HÜLS AKTIENGESELLSCHAFT
- PATENTABTEILUNG -



Verfahren zur Herstellung von 4.4.8.8.10-Pentamethyl-10-cyano-bicyclo-[4.4.0]-decen-(1,6)-on-(2)

Die Erfindung betrifft ein Verfahren zur Herstellung von 4.4.8.8.10-Pentamethyl-10-cyano-bicyclo-[4.4.0]-decen-(1,6)-on-(2) [A] aus einem $C_{15}$-Ketongemisch, wie man es durch Fraktionierung des Sumpfproduktes, das bei der Herstellung von Isophoron, insbesondere bei der Flüssigphasenreaktion, anfällt, bei 1 bis 100 mbar in ca. 30 %iger Ausbeute erhält, durch Addition von Cyanwasserstoff.

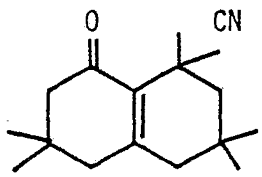

A

Die Verbindung A ist eine geeignete Vorstufe für die Synthese von Aminen, Diaminen, Heterocyclen und eine Vielzahl anderer Stoffe.

Bei der Herstellung von Isophoron durch Kondensation von Aceton entstehen bis zu 10 % höhermolekulare Kondensationsprodukte, die zur Zeit verbrannt werden. Es bestand daher die Aufgabe, dieses Gemisch in Wertprodukten aufzutrennen oder dazu umzusetzen.

Diese Aufgabe wird erfindungsgemäß entsprechend den Angaben der Patentansprüche gelöst.

Überraschenderweise wurde gefunden, daß man durch technisch leicht durchführbare Verfahrensschritte die obige Verbindung A in guter Ausbeute und ausgezeichneter Reinheit erhalten kann, indem man an eine spezielle Fraktion des Isophoron-Sumpfproduktes Cyanwasserstoff addiert.

Durch Fraktionierung des Isophoronsumpfproduktes bei 1 bis 100 mbar, vorzugsweise bei 10 bis 30 mbar erhält man einen Ketonschnitt, der im wesentlichen aus $C_{15}$-Verbindungen besteht. Bei einem Druck von 13 mbar siedet dieser Ketonschnitt, den man in einer Ausbeute von ca. 30 % erhält, bei 125 bis 145 °C. Als Hauptkomponenten enthält dieser Schnitt

die Isomeren B und C

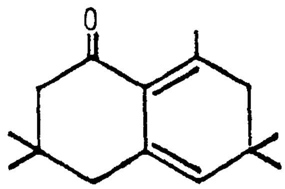

B

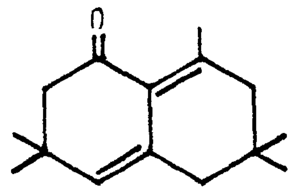

C

Die Lage der Doppelbindungen in B und C ist nicht vollständig abgesichert. Das Verhältnis der beiden Isomeren B und C zueinander ist nicht konstant. Ebenfalls kann der Gehalt der beiden Stoffe in der $C_{15}$-Fraktion unterschiedlich sein. Die Summe beider Isomerer in der $C_{15}$-Fraktion liegt bei 75 bis 90 %.

Die basenkatalysierte Addition von Cyanwasserstoff an den $C_{15}$-Ketonschnitt (Michael-Adititon) ergibt überraschenderweise aus beiden Isomeren B und C das gleiche Nitril A. Weiterhin ist überraschend, daß sich Cyanwasserstoff nicht an andere Verbindung des $C_{15}$-Ketonschnitt addiert und daß auch kein Bis-HCN-Addukt der Struktur D entsteht

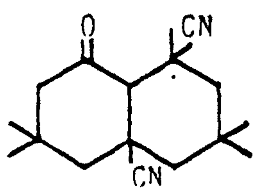

D

Als basische Katalysatoren sind allgemein basische Stoffe geeignet. Bevorzugt setzt man als basische Stoffe Alkalicyanide, Alkalicarbonate, Alkaliphenolate, Alkalialkoholate, insbesondere die Natriumverbindung und besonders bevorzugt das Natriummethylat ein.

Die basenkatalysierte Addition von Cyanwasserstoff erfolgt bei Temperaturen von 50 bis 200 °C, vorzugsweise von 150 bis 170 °C.

Den Cyanwasserstoff setzt man im allgemeinen in Mengen von 5 bis 10 %, bezogen auf den Ketonschnitt, zu.

Statt der aus dem Sumpfprodukt der Isophoronherstellung erhaltenen $C_{15}$-Ketonschnitte mit dem Isomeren B und C kann man natürlich auch an B und/oder C Cyanwasserstoff addieren.

Die Addition von Cyanwasserstoff kann man kontinuierlich oder diskontinuierlich durchführen. Die Addition kann man auch in Gegenwart eines Lösemittels durchführen.

Das Umsetzungsgemisch wird mit Leichtbenzin oder Hexan verdünnt und nach Wäsche mit 1 %iger Säure, vorzugsweise Salpetersäurelösung und Wasser fraktioniert destilliert. Die Fraktionierung erfolgt bevorzugt im Vakuum.

<u>Beispiel</u>

Man fraktioniert das Sumpfprodukt der Isophoronsynthese (durch Kondensation von Aceton) bei einem Druck von 13 mbar. Hierbei erhält man in 30 %iger Ausbeute einen Ketonschnitt ($Kp_{13}$ 125 bis 145 °C), der im wesentlichen aus $C_{15}$-Verbindungen besteht mit den Hauptkomponenten B und C.

In eine 1-l-Rührapparatur mit Bodenventil, ausgerüstet mit Tropftrichter und Rückflußkühler mit angeschlossenem Blasenzähler, wurden 100 g $C_{15}$-Ketongemisch (Summe B,C = 89 %) und 0,5 ml 30 %ige methanolische $NaOCH_3$-Lösung gefüllt. Bei 170 °C wurde mit einer Dosierpumpe eine 30-%ige methanolische $NaOCH_3$-Lösung mit der Geschwindigkeit 0,9 ml/h eingespeist. Zugleich wurde eine Lösung aus 420 g $C_{15}$-Kontongemisch (Summe B,C = 89 %) und 49,2 g HCN zugetropft. Das HCN-Gemisch wurde so schnell zugeführt, wie das HCN abreagierte. (Mit zunehmender Reaktionszeit nahm aufgrund des steigenden Reaktionsvolumen die Umsetzungsgeschwindigkeit zu). Nach 4,5 h war die gesamte Lösung zugetropft. Die Katalysatorzufuhr wurde beendet und der Ansatz 1 h bei 170 °C nachgerührt. Nach dem Abkühlen auf 60 °C wurden 350 ml Hexan zugegeben, und es wurde einmal mit 100 ml 1,5 %iger Salpetersäure und einmal mit 100 ml Wasser gewaschen. Das n-Hexan wurde abgezogen und der Rückstand (576 g) über eine 60 cm lange Gewebepackungskolonne destilliert (Fp = 82 °C).

| Fraktion | Temperatur (° C) Sumpf | Mantel | Kopf | Druck (mbar) | Destillatmenge (g) | % | analytische Zusammensetzung in % Σ BC | Σ U | $C_{16}$-Nitril A |
|---|---|---|---|---|---|---|---|---|---|
| 1 | 153-193 | 70- 80 | 72- 78 | 0,25 | 118,8 | 20,6 | 60 | 40 | 0 |
| 2 | 193-202 | 80-120 | 78-132 | 0,25 | 32,2 | 5,6 | 15 | 68,3 | 16,7 |
| 3 | 202-240 | 120-125 | 132-139 | 0,25-0,3 | 378,2 | 65,7 | - | - | 100 |
| 4 | (240-260) | 125-160 | 139-148 | 0,3 | 31,6 | 5,5 | - | 0,7 | 99,3 |
| Rückstand | | | | | 2,6 | 0,5 | n.a. | n.a. | n.a. |
| Kühlfalle | | | | | 9,8 | 1,7 | n.a. | n.a. | n.a. |

Ausbeute: 92 % d. Th. ber. auf HCN
79 % d. Th. ber. auf Summe B,C,
U = nicht identifizierte Produkte

Patentansprüche:

1. Verfahren zur Herstellung von 4.4.8.8.10-Pentamethyl-10-cyano-bicyclo-[4.4.0]-decen-(1,6)-on-(2), dadurch gekennzeichnet, daß man das Sumpfprodukt, das bei der Herstellung von Isophoron anfällt, bei Drücken von 1 bis 100 mbar fraktioniert, den im wesentlichen aus $C_{15}$-Verbindungen bestehenden Ketonschnitt, der als Hauptkomponenten die Isomeren B und C enthält, isoliert und

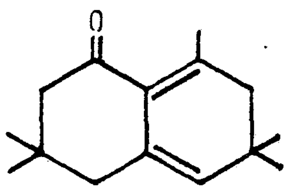

B

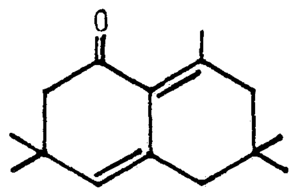

C

in Gegenwart eines basischen Stoffes bei Temperaturen von 50 bis 200 °C Cyanwasserstoff anlagert und das Additionsprodukt nach einer Wäsche fraktioniert destilliert.

2. Verfahren nach Anspruch 1, dadurch gekennzeichnet, daß man als basischen Stoff Alkalicyanide, Alkalicarbonate, Alkaliphenolate und/oder Alkalialkoholate einsetzt, vorzugsweise die Natriumverbindungen.

3. Verfahren nach Anspruch 1 und 2, dadurch gekennzeichnet, daß man als basischen Stoff Natriummethylat einsetzt.

4. Verfahren nach einem der Ansprüche 1 bis 3, dadurch gekennzeichnet, daß man die Anlagerung bei einer Temperatur von 150 bis 170 °C durchführt.

5. Verfahren nach einem der Ansprüche 1 bis 4, dadurch gekennzeichnet, daß man an die Isomeren B und/oder C Cyanwasserstoff anlagert.